# EUROPEAN PATENT SPECIFICATION

(11) **EP 1 044 017 B1**
(45) Date of publication and mention of the grant of the patent: **20.08.2008**
(21) Application number: 98914229.4
(22) Date of filing: 13.03.1998
(51) Int. Cl.: A61K 39/00, A61K 9/20, A61K 31/195, A61K 31/40, A61K 31/415, A61K 38/00, A23L 1/305, A61K 9/50, A61K 31/405, A61K 47/34, A61K 47/38

(54) **SUPPLEMENT FOR DIALYSIS PATIENTS**
DIÄTETISCHE ERGÄNZUNG FÜR DIALYSE PATIENTEN
SUPPLEMENT DIETETIQUE POUR PATIENTS DIALYSES

(30) Priority: 05.01.1998 WO PCT/US98/00014
(43) Date of publication of application: 18.10.2000
(73) Proprietor: THE JOHNS HOPKINS UNIVERSITY, Baltimore, MD 21202 (US)
(72) Inventor: WALSER, Mackenzie, Baltimore, MD 21204 (US)
(74) Representative: Lipscombe, Martin John
(86) International application number: PCT/US1998/003815
(87) International publication number: WO 1999/034813

(56) References cited:
- EP-A- 0 393 781
- EP-A- 0 747 395
- WO-A-94/14430
- FR-A- 2 317 917
- US-A- 3 764 703
- US-A- 4 752 619
- US-A- 4 836 957
- PATENT ABSTRACTS OF JAPAN vol. 0164, no. 49 (C-0986), 18 September 1992 (1992-09-18) & JP 4 159219 A (OTSUKA PHARMACEUT FACTORY INC), 2 June 1992 (1992-06-02)
- PATENT ABSTRACTS OF JAPAN vol. 0154, no. 74 (C-0890), 3 December 1991 (1991-12-03) & JP 3 204814 A (OTSUKA PHARMACEUT FACTORY INC), 6 September 1991 (1991-09-06)
- PATENT ABSTRACTS OF JAPAN vol. 0050, no. 54 (C-050), 15 April 1981 (1981-04-15) & JP 56 008312 A (OTSUKA PHARMACEUT FACTORY INC), 28 January 1981 (1981-01-28)
- FURST et al., "Effects of Nutrition and Catabolic Stress on Intracellular Amino Acid Pools in Uremia", THE AMERICAN JOURNAL OF CLINICAL NUTRITION, Vol. 33, July 1980, pages 1387-1395, XP002914188
- Alvestrand A. et al: (1982) Clin. Nephrology 19:67-73
- Bergström J. et al: (1987) Infusionstherapie 14:8-11
- Kaysen G.A.: (1996) Seminars in Dialysis 9:249-256

## Description

### BACKGROUND

The invention relates to an amino acid supplement for dialysis patients.

Patients on dialysis in the U.S. exhibit a 24% mortality per year. The best predictor of mortality identified to date is the serum albumin concentration. Small differences in serum albumin concentration predict substantial changes in mortality. For example, dialysis patients with a 0.2 g/dl higher serum albumin experience a mortality rate which is 25% lower (Owen et al., NEJM 329: 1001-6, 1993). Hypoalbuminemia is common at the onset of dialysis and during dialysis. It is not amenable to dietary counseling in most cases. Its mechanism is unknown.

It has previously been shown that patients who have been prescribed a very low protein diet supplemented by either essential amino acids or ketoanalogues thereof for at least six months prior to dialysis rarely exhibit hypoalbuminemia at the onset of dialysis (Walser, M., Kidney Internat 44: 1139, 1993), in contrast to the national experience. It has also been shown that such patients have a substantially lower mortality on dialysis, in comparison with the national experience, at least for the first two years, despite consuming the usual dialysis diet (Coresh, J., M. Walser, and S. Hill; J Amer Soc Nephrol 6: 1379, 1995). Whether or not this reduction in mortality can be explained by higher levels of serum albumin could not be ascertained, because these patients were dialyzed at many facilities in several states.

The foregoing observations raise the possibility that supplementation of the diet of the dialysis patients with essential amino acids may protect against hypoalbuminemia, even in patients consuming normal or nearly normal quantities of dietary protein. No mechanism for such an effect is apparent, but similarly, no mechanism for the prevalence of hypoalbuminemia in this population has been identified, although several abnormalities of plasma and intracellular amino acid concentrations have been observed.

There are at least seven small clinical trials of oral supplementation with essential amino acids in dialysis patients reported (1-7). However none of these studies is definitive or well controlled. In reviewing these studies, Kaysen (8) states "Oral nutritional supplementation does not reverse hypoalbuminemia in this patient population"; Wolfson (9) states "However, despite a number of studies (reviewed subsequently) of the use of amino acid supplements, the impact on overall nutritional status has remained controversial"; Ikizler and Hakim (10) state "Furthermore, most of these studies are not controlled and are small in scope and the degree of success is variable".

Intravenous supplementation has been studied more extensively, but the expense of this treatment is prohibitive, and according to Wolfson (9), "... there are numerous flaws in many of these studies".

Furst et al. (11) designed a new formula of essential amino acids, with a higher proportion of valine, lower proportions of leucine and isoleucine, and the inclusion of tyrosine, in an attempt to correct the extracellular and intracellular abnormalities of amino acid concentration that they found in predialysis uremic patients. They have shown that this mixture maintains nitrogen balance while improving abnormalities of amino acid concentrations (11 - 14). This formula was also used in the Feasibility Phase of a large NIH-supported study entitled "Modification of Diet in Renal Disease", and was found to maintain nutrition in these patients with advanced chronic renal failure (15). This mixture has neither been used nor advocated in patients on dialysis.

### SUMMARY OF THE INVENTION

The invention is based on the concept that a mixture of amino acids in tablet form and comprising, in each tablet, L-histidine 45 mg, L-isoleucine 60 mg, L-leucine 90 mg, L-lysine 65 mg, L-methionine 90 mg, L-phenylalanine 70 mg, L-threonine 65 mg, L-tryptophan 25 mg, L-tyrosine 75 mg, and L-valine 135 mg, administered in a dose of 8 to 18 tablets per day, will prevent and/or correct hypoalbuminemia in patients on dialysis (either hemodialysis or peritoneal dialysis), and will therefore improve their survival.

To illustrate the invention, a group of hypoalbuminemic dialysis patients is randomized to receive either these tablets (14 per day) or a similar placebo for three months, in a double-blind fashion, with no change in their diet. Randomization is stratified for two ranges of serum albumin concentration (low and very low) and for hemodialysis vs. peritoneal dialysis. Before supplementation and at monthly intervals during supplementation, serum albumin, anthropometry, and serum amino acid levels are measured. Patients receiving the amino acid supplement should exhibit a greater rise in serum albumin concentration than patients receiving the placebo. Since serum albumin concentration is the best predictor of mortality, patients receiving the amino acid supplement are also anticipated to exhibit lower mortality.

It will be appreciated that the invention contemplates variations in the amounts of amino acids from those recited above. The variation can be up to 30% by weight. However, it is preferred that the indicated ratios of the amino acids be maintained. Thus, for example, the amounts of L-leucine and L-methionine should be about twice the amount of L-histidine administered while the amount of L-isoleucine should be about two-thirds of the L-leucine and L-methionine.

### REFERENCES

1. Phillips ME, Havard J, Howard JP. Oral essential amino acid supplementation in patients on maintenance hemodialysis. Clin Nepbrol 9, 241-248, 1978.
2. Hecking E, Kohler H, Zobel R., Lemmel E-M, Mader H, Opferkuch W, Prellwitz W, Keim HJ, Muller D. Treatment with essential amino acids in patients on chronic hemodialysis; a double blind cross-over study. Am J Clin Nutr 31, 1821-1826, 1978.
3. Ulm A, Neuhauser M, Leber H-W. Influence of essential amino acids and keto acids on protein metabolism and anemia of patients on intermittent hemodialysis, Am J Clin Nutr 31, 1827= -1830, 1978.
4. Counahan R, El-Bishti M, Chantler C. Oral essential amino acids in children on regular hemodialysis. Clin Nephrol 9: 11-14, 1978.
5. Acchiardo S, Moore L, Cockrell S. Effect of essential amino acids on chronic hemodialysis patients. ASAIO Trans 28, 608-614, 1982.
6. Knefati Y, Wone C, Aparicio M. Protein malnutrition of CAPD patients could be treated by oral mixtures of ketoacids and essential amino acids (KA/AA)(Abstract). Kidney Internat 36, Suppl 27, S-303, 1989.
7. Ecder ST, Tuna S, Sever MS, Ozdogan, Ark E, Aysuna N, Bozfakioglu S, Ergin Karadayi H, Aydin AE, Kocak N. Effects of orally administered essential amino acids on patients undergoing maintenance haemodialysis therapy (Abstract) Nephrol Dial Transplant 9; 100, 1994.
8. Kaysen GA. Hypoalbuminemia in dialysis patients. Seminars in Dialysis 9: 249-256, 1996.
9. Wolfson M. Use of nutritional supplements in dialysis patients. Seminars in Dialysis 5: 285-290, 1992.
10. Ikizler TA, Hakim RM. Nutrition in end-stage renal disease. Kidney Internat 50: 343-357, 1996.
11. Furst P, Alvestrand A, Bergstrom J. Effects of nutrition and catabolic stress on intracellular amino acid pools in uremia. Am J Clin Nutr 33: 1387-95, 1980.
12. Alvestrand A, Furst P. Bergstrom J. Plasma and muscle amino acids in uremia: influence of nutrition with amino acids. Clin Nephrol 18: 297-305, 1982.
13. Alvestrand A, Ahlberg M, Furst P, Bergstrom J. Clinical results with a low protein diet and a new amino acid preparation in patients with chronic uremia. Clin Nephrol 19: 67-73, 1983.
14. Bergstrom J, Ahlberg M, Alvestrand A, Furst P. Amino acid therapy for patients with chronic renal failure. Infusionsther Klin Ernahr 14: Suppl 5: 8-11, 1987.
15. Modification of Diet in Renal Disease Study Group. The Modification of Diet in Renal Disease Study: design, methods, and results from the Feasibility Study. Am J Kidney Dis 20: 18-33, 1992.

## Claims

1. Use of a composition comprising, histidine 45 mg, L-isoleucine 60 mg, L-leucine 90 mg, L-lysine 65 mg, L-methionine 90 mg, L-phenylalanine 70 mg, L-threonine 65mg, L-tryptophan 25mg, L-tyrosine 75mg, and L-valine 135 mg, or amounts of any of the foregoing that vary by up to 30% by weight, for the manufacture of a diet supplement for administration to a dialysis patient for the treatment of hypoalbuminemia.

2. Use of a composition in accordance with claim 1 wherein the supplement is in tablet form and administered in a dose of 8-18 tablets per day.

3. Use of a composition in accordance with claim 1 or 2, wherein supplementation of the diet of dialysis patients with essential amino acids may protect against hypoalbuminemia, even in patients consuming normal or nearly normal quantities of dietary protein.

4. Use of a composition in accordance with any one of the preceding claims wherein the diet supplement is for administration to hemodialysis patients.

5. Use of a composition in accordance with any one of the preceding claims wherein the diet supplement is for administration to peritoneal dialysis patients.

6. A composition comprising, histidine 45mg, L-isoleucine 60mg, L-leucine 90mg, L-lysine 65mg, L-methionine 90mg, L-phenylalanine 70mg, L-threonine 65mg, L-tryptophan 25mg, L-tyrosine 75mg, and L-valine 135mg, or amounts of any of the foregoing that vary by up to 30% by weight, for administration as a diet supplement to a dialysis patient for the treatment of hypoalbuminemia.

7. A composition in accordance with claim 6 wherein the supplement is in tablet form and administered in a dose of 8-18 tablets per day.

8. A composition in accordance with claim 6 or 7, wherein supplementation of the diet of dialysis patients with essential amino acids may protect against hypoalbuminemia, even in patients consuming normal or nearly normal quantities of dietary protein.

9. A composition in accordance with any one of claims 6 to 8 wherein the diet supplement is for administration to hemodialysis patients.

10. A composition in accordance with any one of claims 6 to 8 wherein the diet supplement is for administration to peritoneal dialysis patients.

## Patentansprüche

1. Verwendung einer Zusammensetzung, welche 45 mg Histidin, 60 mg L-Isoleucin, 90 mg L-Leucin, 65 mg L-Lysin, 90 mg L-Methionin, 70 mg L-Phenylalanin, 65 mg L-Threonin, 25 mg L-Tryptophan, 75 mg L-Tyrosin und 135 mg L-Valin oder eine um bis zu 30 Ges.-% abweichende Menge eines der vorstehend genannten umfasst, zur Herstellung eines Nahrungsergänzungsmittels zur Verabreichung an einen Dialysepatienten zur Behandlung von Hypoalbuminämie.

2. Verwendung einer Zusammensetzung gemäß Anspruch 1, wobei das Ergänzungsmittel in Tablettenform vorliegt und in einer Dosis von 8 bis 18 Tabletten pro Tag verabreicht wird.

3. Verwendung einer Zusammensetzung gemäß Anspruch 1 oder 2, wobei die Ergänzung der Nahrung von Dialysepatienten mit essentiellen Aminosäuren gegen Hypoalbuminämie schützen kann, sogar bei Patienten, die normale oder nahezu normale Mengen an Nahrungsprotein aufnehmen.

4. Verwendung einer Zusammensetzung gemäß einem der vorstehenden Ansprüche, wobei das Nahrungsergänzungsmittel zur Verabreichung an Hämodialysepatienten ist.

5. Verwendung einer .Zusammensetzung gemäß einem der vorstehenden Ansprüche, wobei das Nahrungsergänzungsmittel zur Verabreichung an Peritonealdialysepatienten ist.

6. Zusammensetzung, welche 45 mg Histidin, 60 mg L-Isoleucin, 90 mg L-Leucin, 65 mg L-Lysin, 90 mg L-Methionin, 70 mg L-Phenylalanin, 65 mg L-Threonin, 25 mg L-Tryptophan, 75 mg L-Tyrosin und 135 mg L-Valin oder eine um bis zu 30 Gew.-% abweichende Menge eines der vorstehend genannten umfasst, zur Verabreichung als Nahrungsergänzungsmittel an einen Dialysepatienten zur Behandlung von Hypoalbuminämie.

7. Zusammensetzung gemäß Anspruch 6, wobei das Ergänzungsmittel in Tablettenform vorliegt und in einer Dosis von 8 bis 18 Tabletten pro Tag verabreicht wird.

8. Zusammensetzung gemäß Anspruch 6 oder 7, wobei die Ergänzung der Nahrung von Dialysepatienten mit essentiellen Aminosäuren gegen Hypoalbuminämie schützen kann, sogar bei Patienten, die normale oder nahezu normale Mengen an Nahrungsprotein aufnehmen.

9. Zusammensetzung gemäß einem der Ansprüche 6 bis 8, wobei das Nahrungsergänzungsmittel zur Verabreichung an Hämodialysepatienten ist.

10. Zusammensetzung gemäß einem der Ansprüche 6 bis 8, wobei das Nahrungsergänzungsmittel zur Verabreichung an Peritonealdialysepatienten ist.

## Revendications

1. Utilisation d'une composition comprenant 45 mg d'histidine, 60 mg de L-isoleucine, 90 mg de L-leucine, 65 mg de L-lysine, 90 mg de L-méthionine, 70 mg de L-phénylalanine, 65 mg de L-thréonine, 25 mg de L-tryptophane, 75 mg de L-tyrosine et 135 mg de L-valine, ou des quantités des éléments cités précédemment qui varient jusqu'à 30 % en poids, pour la fabrication d'un supplément diététique destiné à être administré à un patient sous dialyse pour le traitement de l'hypoalbuminémie.

2. Utilisation d'une composition selon la revendication 1, selon laquelle le supplément est sous forme de comprimé et administré à une dose de 8 à 18 comprimés par jour.

3. Utilisation d'une composition, selon la revendication 1 ou 2, selon laquelle la supplémentation du régime alimentaire de patients sous dialyse en acides aminés essentiels peut protéger contre l'hypoalbuminémie, même dans le cas de patients consommant des quantités normales ou quasi-normales de protéines alimentaires.

4. Utilisation d'une composition selon l'une quelconque des revendications précédentes, selon laquelle le supplément diététique est destiné à être administré à des patients sous hémodialyse.

5. Utilisation d'une composition selon l'une quelconque des revendications précédentes, selon laquelle le supplément diététique est destiné à être administré à des patients sous dialyse péritonéale.

6. Composition comprenant 45 mg d'histidine, 60 mg de L-isoleucine, 90 mg de L-leucine, 65 mg de L-lysine, 90 mg de L-méthionine, 70 mg de L-phénylalanine, 65 mg de L-thréonine, 25 mg de L-tryptophane, 75 mg de L-tyrosine et 135 mg de L-valine, ou des quantités des éléments cités précédemment qui varient jusqu'à 30 % en poids, destinée à être administrée comme supplément diététique à un patient sous dialyse pour le traitement de l'hypoalbuminémie.

7. Composition selon la revendication 6, selon laquelle le supplément est sous forme de comprimé et administré à une dose de 8 à 18 comprimés par jour.

8. Composition selon la revendication 6 ou 7, selon laquelle la supplémentation du régime alimentaire de patients sous dialyse en acides aminés essentiels peut protéger contre l'hypoalbuminémie, même dans le cas de patients consommant des quantités normales ou quasi-normales de protéines alimentaires.

9. Composition selon l'une quelconque des revendications 6 à 8, selon laquelle le supplément diététique est destiné à être administré à des patients sous hémodialyse.

10. Composition selon l'une quelconque des revendications 6 à 8, selon laquelle le supplément diététique est destiné à être administré à des patients sous dialyse péritonéale.
